# EUROPÄISCHE PATENTSCHRIFT

(11) **EP 1 581 165 B1**
(45) Veröffentlichungstag und Bekanntmachung des Hinweises auf die Patenterteilung: **14.03.2007**
(21) Anmeldenummer: 04700120.1
(22) Anmeldetag: 05.01.2004
(51) Int. Cl.: C07H 15/203, A61K 31/7034

(54) **VERBINDUNGEN, DIE FAKTOR VIIA INHIBIEREN**
FACTOR VIIA INHIBITING COMPOUNDS
COMPOSES INHIBANT LE FACTEUR VIIA

(30) Priorität: 03.01.2003 DE 10300049
(43) Veröffentlichungstag der Anmeldung: 05.10.2005
(73) Patentinhaber: NexusPharma Inc., Langhorne, PA 19047-1098 (US)
(72) Erfinder: ECKL, Robert, 81241 München (DE); WEBER, Lutz, 82110 Germering (DE); OEFNER, Christian, 79108 Freiburg (DE)
(74) Vertreter: Held, Stephan
(86) Internationale Anmeldenummer: PCT/EP2004/000026
(87) Internationale Veröffentlichungsnummer: WO 2004/060261

(56) Entgegenhaltungen:
- EP-A- 0 290 321
- EP-A- 0 560 568
- EP-A- 1 078 917
- WO-A-01/90051
- WO-A-02/16312
- WO-A-99/65934

## Beschreibung

Die vorliegende Erfindung betrifft neue Verbindungen mit blutgerinnungshemmender Wirkung, sogenannte Antikoagulantien. Diese Verbindungen stellen sehr wirksame Faktor VIIa-Inhibitoren dar und sind deshalb vor allem bei der Behandlung und/oder Vorbeugung von Thrombosen, Apoplexie, Herzinfarkt, Entzündung, Arteriosklerose und Tumorerkrankungen von Interesse.

Thromboembolytische Erkrankungen beruhen auf einer erhöhten Blutgerinnungsneigung bei Personen mit Risikofaktoren, wie z.B. größeren Operationen, längerer Immobilisierung, Knochenbrüchen der unteren Extremitäten, Fettleibigkeit, Blutfett-Stoffwechselstörungen, Infektionen mit gramnegativen Organismen, Krebs und höherem Alter.

Venöse Thrombosen können dazu führen, daß das von der betroffenen Vene entsorgte Gewebe ein Ödem oder eine Entzündung entwickelt. Thrombose einer tieferliegenden Vene (sogenannte "Deep Vein Thrombosis") kann zu schwerwiegenden Komplikationen wie z.B. Lungenembolie führen. Arterielle Thrombose kann zur ischämischen Nekrose des von der betroffenen Arterie versorgten Gewebes führen, wie z.B. zu myokadialem Infarkt im Falle einer betroffenen Herzkranzarterie. Weitere thromboembolytische Erkrankungen sind z.B. Arteriosklerose, Apoplexie (Schlaganfall), Angina pectoris, Claudicatio intermittens.

Faktor VIIa Inhibitoren hemmen die durch Faktor VIIa und den Gewebefaktor induzierte Bildung der Gerinnungfaktoren Xa, IXa und Thrombin. Sie beeinflussen dadurch sowohl die durch diese Faktoren induzierte Plättchenaggregation als auch die plasmatische Blutgerinnung. Sie verhindern damit die Entstehung von Thromben und können bei der Bekämpfung bzw. Verhütung von Krankheiten, wie Thrombose, Apoplexie, Herzinfakt, Entzündung und Arteriosklerose verwendet werden. Ferner haben diese Verbindungen einen Effekt auf Tumorzellen und verhindern Metastasen. Somit können sie auch als Antitumormittel eingesetzt werden.

WO 0216312 beschreibt Verbindungen die Faktor Xa-Aktivität inhibieren und zur Vorbeugung und/oder Behandlung von thromboembolytischen Erkrankungen verwendet werden können. EP 1 078 917 beschreibt Amidinderivate sowie Arzneimittel, welche dieselben als aktive Inhaltsstoffe enthalten.

Eine Aufgabe der vorliegenden Erfindung bestand in der Bereitstellung neuer Faktor VIIa-Inhibitoren mit verbesserter Wirksamkeit, verringerter Nebenwirkung und/oder erhöhter Selektivität. Zudem sollten geeignete pharmazeutische Zusammensetzungen bereitgestellt werden. Diese Verbindungen bzw. Zusammensetzungen sollten parenteral oder oral, insbesondere oral verabreichbar sein.

Gegenstand der vorliegenden Erfindung ist eine Verbindung der allgemeinen Formel (I): 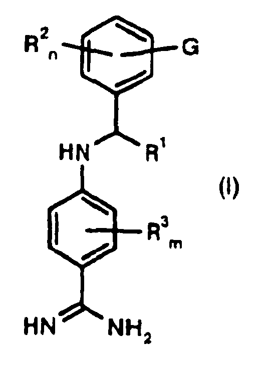 wobei
R¹ ein Wasserstoffatom oder eine Gruppe der Formel COOR⁴ oder CONR⁵R⁶ ist, wobei R⁴, R⁵ und R⁶ unabhängig voneinander Wasserstoffatome, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind, oder R⁵ und R⁶ zusammen Teil eines gegebenenfalls substituierten Heteroaryl- oder Heterocycloalkylrings sind,
die Reste R² unabhängig voneinander Halogenatome, Hydroxy-, Amino-, Nitro- oder Thiolgruppen, Alkyl-, Alkenyl-, Alkinyl-, Heteroalkyl-, Aryl-, Heteroaryl-, Cycloalkyl, Alkylcycloalkyl-, Heteroalkylcycloalkyl-, Heterocycloalkyl-, Aralkyl- oder Heteroaralkylreste sind,
die Reste R³ unabhängig voneinander Halogenatome, Hydroxy-, Amino-, Nitro- oder Thiolgruppen, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind,
G eine Glycosylgruppe ist,
n gleich 0, 1, 2, 3 oder 4 ist und
m gleich 0, 1, 2, 3 oder 4 ist,
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 12 Kohlenstoffatome, vorzugsweise 1 bis 6 Kohlenstoffatome, besonders bevorzugt 1 bis 4 Kohlen-stoffatome aufweist, z.B. die Methyl-, Ethyl-, Propyl-, Isopropyl-, Isobutyl-, tert-Butyl, n-Hexyl-, 2,2-Di-methylbutyl- oder n-Octyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 12 Kohlenstoffatome, vorzugsweise 2 bis 6 Kohlenstoffatome, besonders bevorzugt 2 bis 4 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf gegebenenfalls substituierte Gruppen, bei denen z. B. ein, zwei oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe ausgewählt aus Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}- , R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}- R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₁-C₆-Alkenyl- oder eine C₁-C₆-Alkinylgruppe; R^{b} ein Wasserstoffatom, eine C₁-C₆-Alkyl-, eine C₁-C₆-Alkenyl- oder eine C₁-C₆-Alkinylgruppe; R^{c} ein Wasserstoffatom, eine C₁-C₆-Alkyl-, eine C₁-C₆-Alkenyl- oder eine C₁-C₆-Alkinylgruppe; R^{d} ein Wasserstoffatom, eine C₁-C₆-Alkyl-, eine C₁-C₆-Alkenyl- oder eine C₁-C₆-Alkinylgruppe und Y^{a} eine direkte Bindung, eine C₁-C₆-Alkylen-, eine C₁-C₆-Alkenylen- oder eine C₁-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methylaminomethyl, Ethylaminomethyl, Di-iso-Propyl-aminoethyl, Enolether, Dimethylaminomethyl, Dimethyl-aminoethyl, Acetyl, Propionyl, Butyryloxy, Acetyloxy, Methoxycarbonyl, Ethoxycarbonyl, N-Ethyl-N-Methyl-carbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitril-gruppen.

Der Ausdruck Cycloalkyl bezieht sich auf eine gesättigte oder teilweise ungesättigte (z. B. Cycloalkenyl) cyclische Gruppe, die einen oder mehrere Ringe (bevorzugt 1 oder 2) mit 3 bis 14 Ringkohlenstoffatomen, vorzugsweise 3 bis 10 (insbesondere 3, 4, 5, 6 oder 7) Ringkohlenstoffatome aufweist. Der Ausdruck Cycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]-decanyl-, Norborny-, Cyclohexyl-, Cyclopentenyl-, Cyclo-hexadienyl-, Decalinyl-, Cubanyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind. Beispiele sind die Piperidyl-, Morpholinyl-, Urotropinyl-, Pyrrolidinyl-, Tetrahydrothiophenyl-, Tetrahydropyranyl-, Tetrahydro-furyl-, Oxacyclopropyl-, Azacyclopropyl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkylwie auch Alkyl-, Alkenyl- oder Alkinylgruppen enthalten, z. B. Alkylcycloalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringe mit 3 bis 10 (insbesondere 3, 4, 5, 6 oder 7) Ringkohlenstoffatomen enthält, und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringe mit 3 bis 10 (insbesondere 3, 4, 5, 6 oder 7) Ringatomen und eine oder zwei Alkyl-, Alkenyl-, Alkinyl- oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Hetero-alkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe mit 6 bis 14 Ringkohlenstoffatomen, vorzugsweise 6 bis 10 (insbesondere 6) Ringkohlenstoffatomen enthält. Der Ausdruck Aryl (bzw. Ar) bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe mit 5 bis 14 Ringatomen, vorzugsweise 5 bis 10 (insbesondere 5 oder 6) Ringatomen enthält und ein oder mehrere (bevorzugt 1, 2, 3 oder 4) Sauerstoff-, Stickstoff-, Phosphor- oder Schwefel-Ringatome (bevorzugt O, S oder N) enthält. Der Ausdruck Heteroaryl bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z. B. Arylalkyl-, Arylalkenyl-, Aryl-alkinyl-, Arylcycloalkyl-, Arylcycloalkenyl-, Alkylaryl-cycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthaline, Indanon, Phenylcyclopentyl, Cumol, Cyclo-hexylphenyl, Fluoren und Indan. Bevorzugt enthält eine Aralkylgruppe ein aromatisches Ringsystem (1 oder 2 Ringe) mit 6 bis 10 Ringkohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten. Bevorzugt enthält eine Heteroaralkylgruppe ein aromatisches Ringsystem (1 oder 2 Ringe) mit 5 oder 6 bis 10 Ringkohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocyclo-alkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethyl-indolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf gegebenenfalls substituierte Gruppen, in denen z. B. ein oder mehrere Wasserstoffatome solcher Gruppen durch Fluor-, Chlor-, Brom- oder Jodatome oder OH, =O, SH, =S, NH₂, =NH oder NO₂-Gruppen ersetzt sind.

Der Ausdruch "gegebenenfalls substituiert" bezieht sich auch auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkylgruppen substituiert sind.

Im Kontext der vorliegenden Erfindung bezieht sich der Ausdruck Glycosylgruppe auf ein über eine α- oder β-O, S, N oder C-glycosidische Bindung (bevorzugt eine O-glycosidische Bindung) gebundenes Saccharid (Mono- oder Oligosaccharid), insbesondere ein Monosaccharid, vorzugsweise β-D-Glucose.

Verbindungen der Formel (I) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) sowie Gemische davon umfasst. Des weiteren sind von der vorliegenden Erfindung alle tautomeren Formen der Verbindungen der Formel (I) umfasst.

Bevorzugt sind Verbindungen der Formel (I), wobei R¹ ein Wasserstoffatom oder eine Gruppe der Formel COOR⁴ oder CONR⁵R⁶ ist, wobei R⁴, R⁵ und R⁶ unabhängig voneinander Wasserstoffatome, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind, oder R⁵ und R⁶ zusammen Teil eines gegebenenfalls substituierten Heteroaryl- oder Heterocycloalkylrings sind.

Weiter bevorzugt ist R⁴ ein Wasserstoffatom, ein C₁-C₄ Alkyl oder ein Benzylrest.

Besonders bevorzugt ist R¹ ein Wasserstoffatom oder eine Gruppe der Formel COOH oder COOEt.

Des weiteren bevorzugt ist R¹ eine Gruppe der Formel CONHR⁵, wobei R⁵ wiederum bevorzugt eine Aralkyl-(insbesondere Benzyl-) oder eine Heteroaralkylgruppe ist.

Weiter bevorzugt sind Verbindungen der Formel (I), wobei m gleich 0 ist.

Wiederum bevorzugt ist m gleich 1, wobei R³ besonders bevorzugt eine Hydroxygruppe ist, die in ortho-Position zur Amidinogruppe an den Phenylring gebunden ist.

Des weiteren bevorzugt sind Verbindungen der Formel (I), wobei n gleich 2 ist.

Wiederum bevorzugt sind die Reste R² unabhängig voneinander C₁-C₄ Alkyloxy-, C₁-C₄ Hydroxyalkyloxy- oder Benzyloxygruppen; wobei R² besonders bevorzugt Methoxy- oder Ethyloxygruppen sind.

Besonders bevorzugt sind Verbindungen der allgemeinen Formel (II): 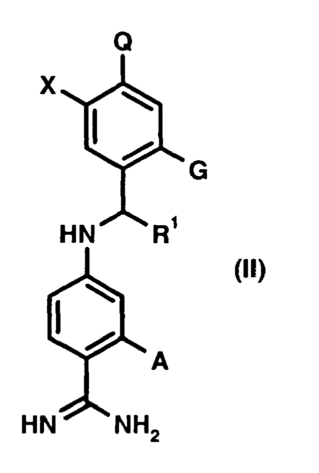 wobei X ein Wasserstoffatom, eine C₁-C₄ Alkyloxy- oder eine Benzyloxygruppe (insbesondere eine Methoxy- oder eine Ethoxygruppe) ist; Q ein Wasserstoffatom, eine C₁-C₄ Alkyloxy- oder eine Benzyloxygruppe (insbesondere eine Methoxy- oder eine Ethoxygruppe) ist; G eine Glycosylgruppe (insbesondere eine β-D-Glucosyloxygruppe) ist; A ein Wasserstoffatom oder eine Hydroxygruppe ist und R¹ ein Wasserstoffatom oder eine Gruppe der Formel COOH oder COOEt ist, oder pharmakologisch akzeptable Salze, Solvate, Hydrate oder pharmakologisch akzeptable Formulierungen derselben.

Insbesondere bevorzugt sind Verbindungen der Formeln (I) und (II), wobei die Stereochemie an dem Kohlenstoffatom, das R¹ trägt (R) nach der Cahn-Ingold-Prelog Nomenklatur aufweist.

Beispiele für pharmakologisch akzeptable Salze der Verbindungen der Formeln (I) oder (II) sind Salze von physiologisch akzeptablen Mineralsäuren wie Salzsäure, Schwefelsäure und Phosphorsäure; oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Verbindungen der Formeln (I) oder (II) können solvatisiert, insbesondere hydratisiert sein. Die Hydratisierung kann z.B. während des Herstellungsverfahrens oder als Folge der hygroskopischen Natur der anfänglich wasserfreien Verbindungen der Formeln (I) oder (II) auftreten.

Die pharmazeutischen Zusammensetzungen gemäß der vorliegenden Erfindung enthalten mindestens eine Verbindung der Formeln (I) oder (II) als Wirkstoff und fakultativ Trägerstoffe und/oder Adjuvantien.

Die Pro-Drugs, die ebenfalls Gegenstand der vorliegenden Erfindung sind, bestehen aus einer Verbindung der Formeln (I) oder (II) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Hydroxy-, Methoxy-, Ethoxy-, Benzyloxy-, Acetyl- oder Acetyloxy-Gruppe.

Eine Verbindung oder pharmazeutische Zusammensetzung der vorliegenden Erfindung kann zur Hemmung von Faktor VIIa-Aktivität, zur Vorbeugung und/oder Behandlung von thromboembolytischen Erkrankungen, arterieller Restenose, Blutvergiftung, Krebs, akuten Entzündungen oder sonstigen Erkrankungen, die durch Faktor VIIa-Aktivität vermittelt werden, und insbesondere von venösen Thrombosen, Ödemen oder Entzündungen, von "Deep Vein Thrombosis", Lungenembolien, thromboembolytischen Komplikationen nach größeren Operationen, bei der Gefäßchirurgie, längerer Immobilisierung, Knochenbrüchen der unteren Extremitäten etc., von arteriellen Thrombosen, insbesondere der Herzkranzgefäße bei myokardialem Infarkt sowie Arteriosklerose, Apoplexie, Angina pectoris, Claudicatio intermittens verwendet werden, um nur einige Indikationen zu nennen.

Wie oben erwähnt, liegt die therapeutische Verwendung der Verbindungen der Formeln (I) oder (II), ihrer pharmakologisch akzeptablen Salze bzw. Solvate und Hydrate sowie Formulierungen und pharmazeutischen Zusammensetzungen im Rahmen der vorliegenden Erfindung.

Auch die Verwendung dieser Wirkstoffe zur Herstellung von Arzneimitteln zur Vorbeugung und/oder Behandlung der beschriebenen Erkrankungen ist Gegenstand der vorliegenden Erfindung. Im allgemeinen werden Verbindungen der Formeln (I) oder (II) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere Wirkstoffe beinhalten, die gewöhnlich zur Vorbeugung und/oder Behandlung von thromboembolytischen Erkrankungen eingesetzt werden wie z.B. Warfarin etc.

Zur Vorbeugung und/oder Behandlung der oben beschriebenen Erkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 0,1 µg bis 20 mg/kg Körpergewicht pro Tag geeignet, wobei eine bevorzugte Dosis 0,5 bis 4 mg/kg pro Tag ist. In geeigneten Fällen kann die Dosis auch unter oder über den oben angegebenen Werten liegen.

Die tägliche Dosis kann beispielsweise in 1, 2, 3 oder 4 Einzeldosen verabreicht werden. Auch ist es möglich, die Dosis für eine Woche als Einzeldosis zu verabreichen.

Die hier beschriebenen Verbindungen der allgemeinen Formeln (I) und (II) zeichnen sich gegenüber den im Stand der Technik beschriebenen Verbindungen (EP0921116, WO0035858, W00190051) durch eine geringere Toxizität, verbesserte Wirkung, verbessertes Transportverhalten und eine bessere Bioverfügbarkeit (insbesondere orale Bioverfügbarkeit) aus.

Verbindungen der Formeln (I) und (II) können analog zu den in EP0921116, WO0035858, WO03064440, WO03064378 und WO0190051 beschriebenen Verfahren unter Verwendung geeigneter Ausgangsmaterialien hergestellt werden.

Glycosylierte Benzaldehyde können z. B. nach den in Kleine et al. Carbohydrate Research 1985, 142, 333-337 und Brewster et al. Tetrahedron Letters 1979, 5051-5054 beschriebenen Verfahren hergestellt werden.

### BEISPIELE

Die glycosylierten Benzaldehyde wurden nach den in Kleine et al. Carbohydrate Research 1985, 142, 333-337 beschriebenen Verfahren hergestellt. Diese wurden anschliessend nach folgender allgemeiner Arbeitsvorschrift umgesetzt (WO03064440, WO03064378): 1 mmol Amin und 1 mmol Aldehyd werden in 20 ml Acetonitril/Wasser (Mischungsverhältnis von 1:0 bis 1:1) 30 min bei Raumtemperatur gerührt. Anschließend wird 1 mmol Isonitril zugegeben und weitere 15h gerührt. Das Lösungsmittel wird im Vakuum entfernt, die Acetylgruppen mit 2M NH₃ in Methanol abgespalten und der Rückstand mittels HPLC gereinigt. Die Identifizierung der Verbindungen erfolgte mittels MS.

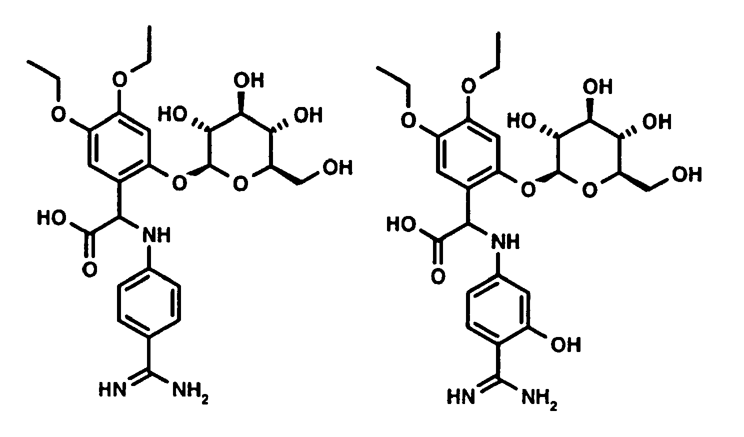

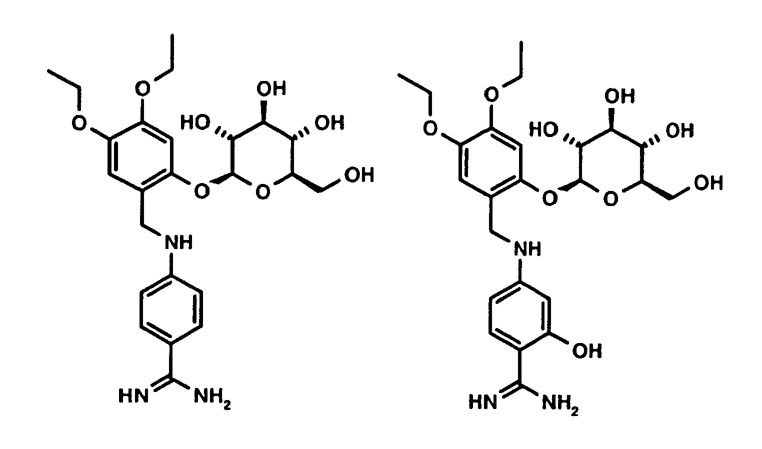

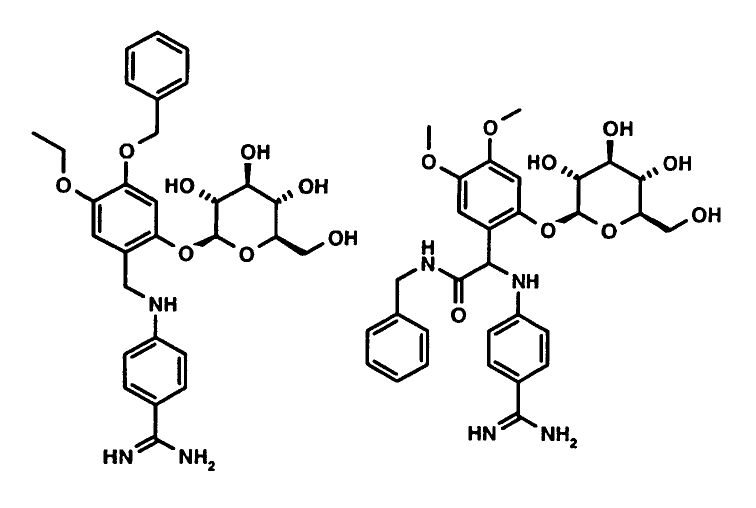

## Patentansprüche

1. Verbindungen der allgemeinen Formel (I): 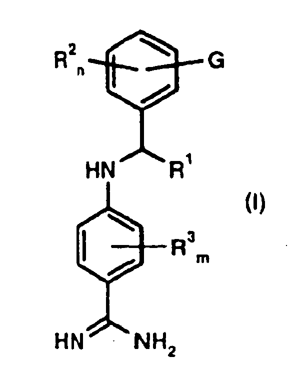 wobei
die Reste R² unabhängig voneinander Halogenatome, Hydroxy-, Amino-, Nitro- oder Thiolgruppen, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind,
die Reste R³ unabhängig voneinander Halogenatome, Hydroxy-, Amino-, Nitro- oder Thiolgruppen, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind,
R¹ ein Wasserstoffatom oder eine Gruppe der Formel COOR⁴ oder CONR⁵R⁶ ist, wobei R⁴, R⁵ und R⁶ unabhängig voneinander Wasserstoffatome, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder Heteroaralkylreste sind, oder R⁵ und R⁶ zusammen Teil eines gegebenenfalls substituierten Heteroaryl- oder Heterocycloalkylrings sind>
G eine Glycosylgruppe ist,
n gleich 0, 1, 2, 3 oder 4 ist und
m gleich 0, 1, 2, 3 oder 4 ist,
oder ein pharmakologisch akzeptables Salz, Solvat, Hydrat oder eine pharmakologisch akzeptable Formulierung derselben.
Der Ausdruck Alkyl bezieht sich auf eine gesättigte, geradkettige oder verzweigte Kohlenwasserstoffgruppe, die 1 bis 12 Kohlenstoffatome aufweist.
Die Ausdrücke Alkenyl und Alkinyl beziehen sich auf zumindest teilweise ungesättigte, geradkettige oder verzweigte Kohlenwasserstoffgruppen, die 2 bis 12 Kohlenstoffatome aufweisen.
Des weiteren beziehen sich die Begriffe Alkyl, Alkenyl und Alkinyl auf gegebenenfalls substituierte Gruppen, bei denen z. B. ein, zwei oder mehrere Wasserstoffatome durch ein Halogenatom ersetz sind.
Der Ausdruck Heteroalkyl bezieht sich auf eine Alkyl-, eine Alkenyl- oder eine Alkinyl-Gruppe, in der ein oder mehrere Kohlenstoffatome durch ein Sauerstoff-, Stickstoff-, Phosphor-, Bor-, Selen-, Silizium- oder Schwefelatom ersetzt sind. Der Ausdruck Heteroalkyl bezieht sich des weiteren auf eine Carbonsäure oder eine von einer Carbonsäure abgeleitete Gruppe-ausgewählt aus Acyl, Acylalkyl, Alkoxycarboxyl, Acyloxy, Acyloxyalkyl, Carboxyalkylamid oder Alkoxycarbonyloxy.
Der Ausdruck Cycloalkyl bezieht sich auf eine gesättigte oder teilweise ungesättigte cyclische Gruppe, die einen oder mehrere Ringe mit 3 bis 14 Ringkohlenstoffatomen aufweist. Der Ausdruck Cycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind.
Der Ausdruck Heterocycloalkyl bezieht sich auf eine Cycloalkylgruppe wie oben definiert, in der ein oder mehrere Ring-Kohlenstoffatome durch ein Sauerstoff-, Stickstoff-, Silizium-, Selen-, Phosphor- oder Schwefelatom ersetzt sind.
Der Ausdruck Heterocycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind.
Der Ausdruck Alkylcycloalkyl bezieht sich auf Gruppen, die entsprechend den obigen Definitionen sowohl Cycloalkylwie auch Alkyl-, Alkenyl- oder Alkinylgruppen enthalten.
Der Ausdruck Heteroalkylcycloalkyl bezieht sich auf Alkylcycloalkylgruppen, wie oben definiert, in der ein oder mehrere Kohlenstoffatome durch ein Sauerstoff-, Stickstoff-, Silizium-, Selen-, Phosphor- oder Schwefelatom ersetzt sind.
Der Ausdruck Aryl bzw. Ar bezieht sich auf eine aromatische Gruppe, die einen oder mehrere Ringe mit 6 bis 14 Ringkohlenstoffatomen enthält. Der Ausdruck Aryl (bzw. Ar) bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind.
Der Ausdruck Heteroaryl bezieht sich auf eine aromatische Gruppe, die einen oder mehrere Ringe mit 5 bis 14 Ringatomen enthält und ein oder mehrere Sauerstoff-, Stickstoff-, Phosphor- oder Schwefel-Ringatome enthält. Der Ausdruck Heteroaryl bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind.
Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten.
Der Ausdruck Heteroaralkyl bezieht sich auf eine Aralkylgruppe wie oben definiert, in der ein oder mehrere Kohlenstoffatome durch ein Sauerstoff-, Stickstoff-, Silizium-, Selen-, Phosphor-, Bor- oder Schwefelatom ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten.
Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf gegebenenfalls substituierte Gruppen,
Der Ausdruch "gegebenenfalls substituiert" bezieht sich auch auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkylgruppen substituiert sind.
Der Ausdruck Glycosylgruppe bezieht sich auf ein über eine α- oder β-O, S, N oder C-glycosidische Bindung gebundenes Saccharid.

2. Verbindungen nach Anspruch 1, wobei R⁴ ein Wasserstoffatom, ein C₁-C₄ Alkyl oder ein Benzylrest ist.

3. Verbindungen nach einem der Ansprüche 1 bis 2, wobei R¹ ein Wasserstoffatom oder eine Gruppe der Formel COOH oder COOEt ist.

4. Verbindungen nach einem der Ansprüche 1 bis 2, wobei R¹ eine Gruppe der Formel CONHR⁵ ist, wobei R⁵ wie in einem der vorstehenden Ansprüche definiert ist.

5. Verbindungen nach einem der Ansprüche 1 bis 4, wobei m gleich 0 ist.

6. Verbindungen nach einem der Ansprüche 1 bis 4, wobei m gleich 1 ist und R³ eine Hydroxygruppe ist, die in ortho-Position zur Amidinogruppe an den Phenylring gebunden ist.

7. Verbindungen nach einem der Ansprüche 1 bis 6, wobei n gleich 2 ist.

8. Verbindungen nach einem der Ansprüche 1 bis 7, wobei die Reste R² unabhängig voneinander C₁-C₄ Alkyloxy-, C₁-C₄ Hydroxyalkyloxy- oder Benzyloxygruppen sind.

9. Pharmazeutische Zusammensetzungen, die eine Verbindung nach den Ansprüchen 1 bis 8 und fakultativ Trägerstoffe und/oder Adjuvanzien enthalten.

10. Verwendung einer Verbindung oder einer pharmazeutischen Zusammensetzung nach einem der Ansprüche 1 bis 9 zur Hemmung von Faktor VIIa.

11. Verwendung einer Verbindung oder einer pharmazeutischen Zusammensetzung nach einem der Ansprüche 1 bis 9 zur Herstellung eines Medikaments zur Behandlung und/oder Vorbeugung von thromboembolytischen Erkrankungen, arterieller Restenose, Blutvergiftung, Krebs, akuten Entzündungen, oder sonstigen Erkrankungen, die durch Faktor VIIa-Aktivität vermittelt werden.

12. Verwendung einer Verbindung oder einer pharmazeutischen Zusammensetzung nach einem der Ansprüche 1 bis 9 zur Herstellung eines Medikaments zum Einsatz bei der Gefäßchirurgie.

## Claims

1. Compounds of the general formula (I): 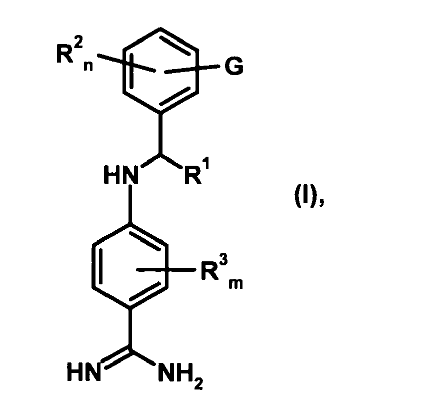 wherein
the radicals R², each independently of any other(s), are halogen atoms, hydroxy, amino, nitro or thiol groups, alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl radicals,
the radicals R³, each independently of any other(s), are halogen atoms, hydroxy, amino, nitro or thiol groups, alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl radicals,
R¹ is a hydrogen atom or a group of formula COOR⁴ or CONR⁵R⁶ wherein R⁴, R⁵ and R⁶ are, each independently of the others, hydrogen atoms, alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl radicals, or R⁵ and R⁶ together are part of an optionally substituted heteroaryl or heterocycloalkyl ring.
G is a glycosyl group,
n is 0, 1, 2, 3 or 4 and
m is 0, 1, 2, 3 or 4,
or a pharmacologically acceptable salt, solvate, hydrate or pharmacologically acceptable formulation thereof.
The expression alkyl refers to a saturated, straight-chain or branched hydrocarbon group having from 1 to 12 carbon atoms.
The expressions alkenyl and alkynyl refer to at least partially unsaturated, straight-chain or branched hydrocarbon groups having from 2 to 12 carbon atoms.
Furthermore, the terms alkyl, alkenyl and alkynyl refer to optionally substituted groups in which e.g. one, two or more hydrogen atoms have been replaced by a halogen atom.
The expression heteroalkyl refers to an alkyl, alkenyl or alkynyl group in which one or more carbon atoms have been replaced by an oxygen, nitrogen, phosphorus, boron, selenium, silicon or sulphur atom. The expression heteroalkyl furthermore refers to a carboxylic acid or to a group derived from a carboxylic acid, selected from acyl, acylalkyl, alkoxycarbonyl, acyloxy, acyloxyalkyl, carboxyalkylamide or alkoxycarbonyloxy.
The expression cycloalkyl refers to a saturated or partially unsaturated cyclic group having one or more rings containing from 3 to 14 ring carbon atoms. The expression cycloalkyl refers furthermore to groups in which one or more hydrogen atoms have been replaced by fluorine, chlorine, bromine or iodine atoms or by OH, =O, SH, =S, NH₂, =NH or NO₂ groups.
The expression heterocycloalkyl refers to a cycloalkyl group as defined above in which one or more ring carbon atoms have been replaced by an oxygen, nitrogen, silicon, selenium, phosphorus or sulphur atom. The expression heterocycloalkyl refers furthermore to groups in which one or more hydrogen atoms have been replaced by fluorine, chlorine, bromine or iodine atoms or by OH, =O, SH, =S, NH₂, =NH or NO₂ groups.
The expression alkylcycloalkyl refers to groups containing both cycloalkyl and also alkyl, alkenyl or alkynyl groups in accordance with the above definitions.
The expression heteroalkylcycloalkyl refers to alkylcycloalkyl groups as defined above in which one or more carbon atoms have been replaced by an oxygen, nitrogen, silicon, selenium, phosphorus or sulphur atom.
The expression aryl or Ar refers to an aromatic group which has one or more rings containing from 6 to 14 ring carbon atoms. The expression aryl (or Ar) refers furthermore to groups in which one or more hydrogen atoms have been replaced by fluorine, chlorine, bromine or iodine atoms or by OH, SH, NH₂ or NO₂ groups.
The expression heteroaryl refers to an aromatic group which has one or more rings containing from 5 to 14 ring atoms, and containing one or more oxygen, nitrogen, phosphorus or sulphur ring atoms. The expression heteroaryl refers furthermore to groups in which one or more hydrogen atoms have been replaced by fluorine, chlorine, bromine or iodine atoms or by OH, SH, NH₂ or NO₂ groups.
The expression aralkyl refers to groups containing both aryl and also alkyl, alkenyl, alkynyl and/or cycloalkyl groups in accordance with the above definitions.
The expression heteroaralkyl refers to an aralkyl group as defined above in which one or more carbon atoms have been replaced by an oxygen, nitrogen, silicon, selenium, phosphorus, boron or sulphur atom, that is to say to groups containing both aryl or heteroaryl and also alkyl, alkenyl or alkynyl and/or heteroalkyl and/or cycloalkyl and/or heterocycloalkyl groups in accordance with the above definitions.
The expressions cycloalkyl, heterocycloalkyl, alkylcycloalkyl, heteroalkylcycloalkyl, aryl, heteroaryl, aralkyl and heteroaralkyl also refer to optionally substituted groups.
The expression "optionally substituted" also refers to groups in which one or more hydrogen atoms have been replaced by fluorine, chlorine, bromine or iodine atoms or by OH, =O, SH, =S, NH₂, =NH or NO₂ groups. The expression refers furthermore to groups which are substituted by unsubstituted C₁-C₆alkyl, C₂-C₆alkenyl, C₂-C₆alkynyl, C₁-C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.
The expression glycosyl group refers to a saccharide bonded by way of an α- or β-O-, -S-, -N- or -C-glycosidic bond.

2. Compounds according to claim 1, wherein R⁴ is a hydrogen atom, a C₁-C₄alkyl or benzyl radical.

3. Compounds according to one of claims 1 to 2, wherein R¹ is a hydrogen atom or a group of formula COOH or COOEt.

4. Compounds according to one of claims 1 to 2, wherein R¹ is a group of formula CONHR⁵ wherein R⁵ is defined as in one of the preceding claims.

5. Compounds according to one of claims 1 to 4, wherein m is 0.

6. Compounds according to one of claims 1 to 4, wherein m is 1 and R³ is a hydroxy group which is bonded to the phenyl ring in a position ortho to the amidino group.

7. Compounds according to one of claims 1 to 6, wherein n is 2.

8. Compounds according to one of claims 1 to 7, wherein the radicals R², each independently of any other(s), are C₁-C₄alkyloxy, C₁-C₄hydroxyalkyloxy or benzyloxy groups.

9. Pharmaceutical compositions comprising a compound according to claims 1 to 8 as active ingredient and, optionally, carrier substances and/or adjuvants.

10. Use of a compound or of a pharmaceutical composition according to one of claims 1 to 9 in inhibiting factor VIIa.

11. Use of a compound or of a pharmaceutical composition according to one of claims 1 to 9 in the preparation of a medicament for the treatment and/or prevention of thromboembolic conditions, arterial restenosis, septicaemia, cancer, acute inflammation or other conditions mediated by factor Vila activity.

12. Use of a compound or of a pharmaceutical composition according to one of claims 1 to 9 in the preparation of a medicament for utilisation in vascular surgery.

## Revendications

1. Composés des formules générales ( I ) :
Où 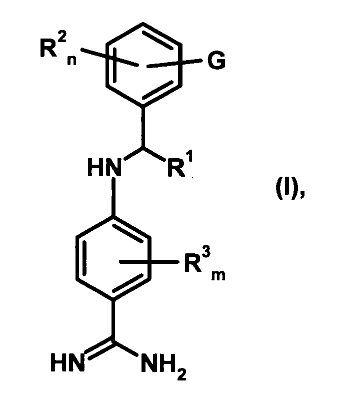 les radicaux R², chacun indépendamment, représentent des atomes d'halogène, des groupes hydroxy, des groupes amino, des groupes nitro ou des groupes thiol, des radicaux alkyle, alkényle, alkinyle, hétéroalkyle, aryle, hétéroaryle, cycloalkyle, alkylcycloalkyle, hétéroalkyle, cycloalkyle, hétérocycloalkyle, aralkyle ou des radicaux hétéroaralkyle,
les radicaux R³, chacun indépendamment, représentent des atomes d'halogène, des groupes hydroxy, des groupes amino, des groupes nitro ou des groupes thiol, des radicaux alkyle, alkényle, alkinyle, hétéroalkyle, aryle, hétéroaryle, cycloalkyle, alkylcycloalkyle, hétéroalkyle, cycloalkyle, hétérocycloalkyle, aralkyle ou des radicaux hétéroaralkyle,
R¹ représente un atome d'hydrogène ou un groupe de la formule COOR⁴ ou CONR⁵R⁶, où R⁴, R⁵, R⁶, chacun indépendamment, sont des atomes d'hydrogène, des radicaux alkyle, alkényle, alkinyle, hétéroalkyle, aryle, hétéroaryle, cycloalkyle, alkylcycloalkyle, hétéroalkylcycloalkyle, hétérocycloalkyle, aralkyle ou hétéroaralkyle, ou R5 et R6 ensembles, sont une partie d'un cycle hétéroaryle ou hétérocycloalkyle éventuellement substitué.
G est un groupe de glycosyle
n est aussi 0, 1, 2, 3 ou 4 et
m est aussi 0, 1, 2, 3 ou 4,
ou un sel, un solvate, un hydrate pharmaceutique acceptable ou une formulation acceptable sur le plan pharmaceutique de celui-ci.
L'expression alkyle représente un groupe d'hydrocarbure saturé, linéaire ou ramifié, comportant de 1 à 12 atomes de carbone.
Les expressions alkényle et alkinyle représentent des groupes d'hydrocarbure au moins partiellement non-saturés, linéaires ou ramifiés, comportant de 2 à 12 atomes de carbone.
De plus, les termes alkyle, alkényle et alkinyle représentent des groupes éventuellement substitués, pour lesquels par exemple, un, deux ou plusieurs atomes d'hydrogènes sont remplacés par un atome d'halogène.
L'expression hétéroalkyle représente un groupe d'alkyle, d'alkényle ou d'alkinyle, dans lequel un ou plusieurs atomes de carbone sont remplacés par un atome d'oxygène, d'azote, de phosphore, de bore, de sélénium, de silicium ou de soufre. L'expression hétéroalkyle représente de plus un acide de carbone ou un groupe dérivé d'un acide de carbone, choisi parmi l'acyle, l'acylalkyle, l'alkoxycarbonyle, l'acyloxy, l'acyloxyalkyle, l'amide carboxyalkyle ou l'alkoxycarbonyloxy.
L'expression cycloalkyle représente un groupe cyclique saturé ou partiellement non-saturé, comportant un ou plusieurs cycles ayant de 3 à 14 atomes de carbone cycliques. L'expression cycloalkyle représente de plus des groupes, pour lesquels un ou plusieurs atomes d'hydrogène sont remplacés par des atomes de fluor, de chlore, de brome ou d'iode ou des groupes OH, =O, SH, =S, NH₂, =NH ou NO₂.
L'expression hétérocycloalkyle représente un groupe cycloalkyle, comme défini ci-dessus, dans lequel un ou plusieurs atomes de carbone cycliques sont remplacés par un atome d'oxygène, d'azote, de silicium, de sélénium, de phosphore ou de soufre.
L'expression hétérocycloalkyle représente de plus des groupes, dans lesquels un ou plusieurs atomes d'hydrogène sont remplacés par des atomes de fluor, de chlore, de brome ou d'iode ou des groupes OH, =O, SH, =S, NH₂, =NH ou NO₂
L'expression alkylcycloalkyle représente des groupes, conformément aux définitions ci-dessus, comportent aussi bien des groupes cycloalkyle que des groupes alkyle, alkenyle ou alkinyle.
L'expression hétéroalkylcycloalkyle représente des groupes alkylcycloalkyle, comme défini ci-dessus, dans lesquels un ou plusieurs atomes de carbone sont remplacés par des atomes d'oxygène, d'azote, de silicium, de sélénium de phosphore ou de soufre.
L'expression aryle, ou encore Ar, représente un groupe aromatique, comportant un ou plusieurs cycles ayant de 6 à 14 atomes de carbone cycliques. De plus, l'expression aryle ou encore Ar, représente des groupes, dans lesquels un ou plusieurs atomes d'hydrogène sont remplacés par des atomes de fluor, de chlore, de brome ou d'iode ou des groupes OH, SH, NH₂, ou NO₂.
L'expression hétéroaryle représente un groupe aromatique, comportant un ou plusieurs cycles ayant de 5 à 14 atomes cycliques et un ou plusieurs atomes cycliques d'oxygène, d'azote, de phosphore ou de soufre. L'expression hétéroaryle représente de plus des groupes, dans lesquels un ou plusieurs atomes d'hydrogène sont remplacés par des atomes de fluor, de chlore, de brome ou d'iode ou des groupes OH, SH, NH₂ ou NO₂.
L'expression aralkyle représente des groupes qui, conformément aux définitions ci-dessus, comportent aussi bien des groupes aryle que des groupes alkyle, alkényle, alkinyle et/ou cycloalkyle.
L'expression hétéroaralkyle représente un groupe aralkyle comme défini ci-dessus, dans lequel un ou plusieurs atomes de carbone sont remplacés par un atome d'oxygène, d'azote, de silicium, de sélénium, de phosphore, de bore ou de soufre, ce qui signifie des groupes qui, conformément aux définitions ci-dessus, comportent aussi bien des groupes aryle, voire hétéroaryle, que des groupes alkyle, alkényle, alkinyle et/ou hétéroalkyle et/ou cycloalkyle et/ou hétérocycloalkyle.
Les expressions cycloalkyle, hétérocycloalkyle, alkylcycloalkyle, hétéroalkylcycloalkyle, aryle, hétéroaryle, aralkyle et hétéroaralkyle représentent aussi des groupes éventuellement substitués.
L'expression « éventuellement substituée » représente des groupes, dans lesquels un ou plusieurs atomes d'hydrogène sont remplacés par des atomes de fluor, de chlore, de brome ou d'iode ou des groupes OH, =O, SH, =S, NH₂, =NH ou NO₂. De plus cette expression représente des groupes substitués par des groupes C₁-C₆ alkyle, C₂-C₆ alkényle, C₂-C₆ alkinyle, C₁-C₆ hétéroalkyle, C₃-C₁₀ cycloalkyle, C₂-C₉ hétérocycloalkyle, C₆-C₁₀ aryle, C₁-C₉ hétéroaryle, C₇-C₁₂ aralkyle ou C₂-C₁₁ hétéroaralkyle non-substitués.
L'expression groupe de glycosyle représente un saccharide lié par une liaison glucosidique de la façon α- ou β-O-, -S-, -N- ou -C-.

2. Composés selon la revendication 1, où R⁴ est un atome d'hydrogène ou un radical d'alkyle C₁-C₄ ou de benzyle

3. Composés selon l'une des revendications 1 à 2, où R¹ est un atome d'hydrogène ou un groupe de la formule COOH ou COOEt.

4. Composés selon une des revendications 1 à 2, où R¹ représente un groupe de la formule CONHR⁵, où R⁵ est défini comme dans l'une des revendications précédentes.

5. Composés selon une des revendications 1 à 4, où m est 0

6. Composés selon une des revendications 1 à 4, où m est 1 et R³ est un groupe hydroxy, associé au cycle phényle en position ortho par rapport au groupe amidine.

7. Composés selon une des revendications 1 à 6, où n est 2

8. Composés selon une des revendications 1 à 7, où les radicaux R², chacun indépendamment, sont des groupes C₁-C₄ alkyloxy, C₁-C₄ hydroxyalkyloxy ou benzyloxy.

9. Composition pharmaceutique, comportant un composé selon les revendications 1 à 8 et facultativement des éléments de support ou des adjuvants.

10. Utilisation d'un composé ou d'une composition pharmaceutique selon une des revendications 1 à 9, pour l'inhibition de facteurs VIIa.

11. Utilisation d'un composé ou d'une composition pharmaceutique selon une des revendications 1 à 9, pour la préparation d'un médicament et/ou préventif contre les maladies thromboemboliques, la resténose artérielle, les infections sanguines, le cancer, les inflammations aiguës et d'autres maladies liées à l'activité du facteur VIIa.

12. Utilisation d'un composé ou d'une composition pharmaceutique selon une des revendications 1 à 9, pour la préparation d'un médicament pouvant servir en chirurgie vasculaire.
